(19) Europäisches Patentamt
European Patent Office
Office européen des brevets

(11) Veröffentlichungsnummer: **0 291 873 B1**

(12) **EUROPÄISCHE PATENTSCHRIFT**

(45) Veröffentlichungstag der Patentschrift: **10.03.93**

(51) Int. Cl.$^5$: **C12Q 1/56**

(21) Anmeldenummer: **88107706.9**

(22) Anmeldetag: **13.05.88**

# (54) Verfahren zum Nachweis der antiaggregatorischen Wirkung von vasoaktiven Stoffen, speziell von Phosphodiesterase- und/oder Cyclooxygenase-Hemmern.

(30) Priorität: **22.05.87 DE 3717337**

(43) Veröffentlichungstag der Anmeldung:
**23.11.88 Patentblatt 88/47**

(45) Bekanntmachung des Hinweises auf die Patenterteilung:
**10.03.93 Patentblatt 93/10**

(84) Benannte Vertragsstaaten:
**AT BE CH DE ES FR GB GR IT LI LU NL SE**

(56) Entgegenhaltungen:
**EP-A- 0 169 465**
**EP-A- 0 208 962**
**WO-A-87/00434**
**US-A- 4 051 236**

**NATURE, Band 279, Nr. 5716, 28. Juni 1979, Seiten 799-800, Basingstoke; M. CHIGNARD et al.;**

**DRUGS OF THE FUTURE, Band 11, Nr. 8, 1986, Seiten 689-701, Barcelona; M. KUCHAR et al.;**

(73) Patentinhaber: **HOECHST AKTIENGESELLSCHAFT**
**Postfach 80 03 20**
**W-6230 Frankturt am Main 80(DE)**

(72) Erfinder: **Weithmann, Klaus Ulrich, Dr.**
**Am Domherrnwald 18**
**W-6238 Hofheim am Taunus(DE)**
Erfinder: **Seiffge, Dirk, Dr.**
**Hauptstr. 2**
**W-6309 Münzenberg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

## Beschreibung

Die Bildung von Thrombozytenaggregaten an der Blutgefäßwand wird als wichtiger Schritt in der Pathogenese von thrombotischen Krankheiten angesehen. Die biochemischen Reaktionswege, die zur Aggregation der Thrombozyten führen, sind bereits gut untersucht und beschrieben worden.

Die Thrombozytenaggregation kann durch gewisse Stimulatoren ausgelöst werden, z.B. durch Arachidonsäure $CH_3(CH_2)_4(CH=CHCH_2)_4(CH_2)_2COOH$. Diese mehrfach ungesättigte Fettsäure wird in den Thrombozyten durch das Enzym Cyclooxygenase zum Prostaglandin-Endoperoxid und weiter zum Thromboxan $A_2$ umgewandelt, die beide äußerst wirksame Induktoren der Thrombozytenaggregation darstellen. Die Thrombozytenaggregation kann auch durch andere Substanzen induziert werden, die, wie Collagen, z.B. die Freisetzung von intrazellulärer Arachidonsäure aus Lipidfraktionen der Thrombozyten katalysieren.

Mit Hilfe von Hemmstoffen des Cyclooxygenase-Enzyms kann die induzierte Aggregation der Thrombozyten inhibiert werden. Derartige Hemmstoffe sind bereits bekannt. Die experimentell und klinisch nachweisbaren aggregationshemmenden und antithrombotischen Effekte der O-Acetylsalicylsäure (Acetylsalicylsäure) werden mit ihrer Hemmwirkung auf die Cyclooxygenase erklärt.

Auch verschiedenen Vertretern aus der Substanzklasse der Xanthine und der Pyrimido-Pyrimidine werden aggregationshemmende und antithrombotische Wirkungen zugeschrieben. Typische Beispiele sind Pentoxifyllin bzw. Dipyridamol; das sind die Verbindung der Formeln

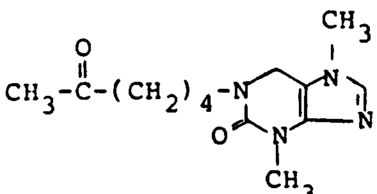

und

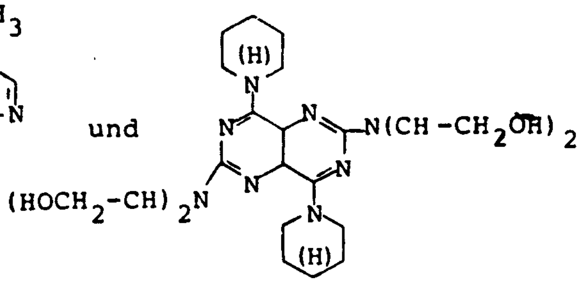

(Pentoxifyllin) (Dipyridamol)

Diese Substanzen sind als Hemmstoffe der Thrombozyten-Phosphodiesterase beschrieben worden. Dieses Enzym katalysiert die Hydrolyse des cyclischen Adenosinmonophospates (cyclo AMP) zum inaktiven Folgeprodukt 5′-AMP. Durch Inhibierung des Enzyms wird der Abbau des cyclo AMP gehemmt. Es kann somit zu einem intrathrombozytären cyclo AMP-Anstieg kommen, der zu einer Hemmung der Thrombozytenaggregation führt.

Die aggregationshemmende Wirkung einer weiteren Substanzklasse, der Prostaglandine, wird ebenfalls mit Hilfe von cyclo AMP-stimulierenden Mechanismen erklärt. Ein Vertreter dieser Substanzklasse ist z.B. das Prostaglandin $I_2$ ($PGI_2$), das als wichtiger Metabolit der Arachidonsäure in der Blutgefäßwand beschrieben worden ist.

Für den Nachweis der aggregationshemmenden Wirkung der Xanthine sowie der Pyrimido-Pyrimidine in vitro, aber auch der Cyclooxygenase-Hemmer, wie der Acetylsalicylsäure, sind ziemlich hohe Wirkstoff-Konzentrationen erforderlich.

Der Wirkungsnachweis am Tier kann auf verschiedene Weise, nämlich durch Untersuchung des Blutes ex vivo oder in vivo, durch geeignete invasive Techniken, erfolgen. Im ex vivo Experiment wird dem Tier nach Applikation der Wirksubstanz, die auf übliche Weise, z.B. per os oder intravenös, erfolgen kann, Blut entnommen und außerhalb des Tierkörpers die Thrombozytenaggregation mechanisch bzw. mit Hilfe der bekannten Induktoren induziert. Als Meßgröße wird die Aggregationsneigung (Aggregabilität) der Thrombozyten bestimmt; geeignete Meßmethoden sind z.B. beschrieben von G.V.R. Born in Nature Vol. 194 (1962), 927 (optische Messung in Blutplasma) und von C. Ingerman-Wolenski et al. in J. Lab. Clin. Med., January 1983, Vol. 101 No. 1, 44 (Messung des elektrischen Widerstandes in Vollblut). Diese ex vivo Technik läßt sich problemlos auch auf Untersuchungen am Menschen übertragen. Allerdings ist es hierbei von großem Nachteil, daß die aggregationshemmende Wirkung der Medikamente erst nach relativ hoher Dosierung nachgewiesen werden kann.

Mit Hilfe von invasiven Techniken kann die erwünschte Wirkung der Medikamente zwar nach geringerer Dosierung in vivo nachgewiesen werden. Aus naheliegenden Gründen können aber diese Techniken, zu

denen beispielsweise die Induktion von Thrombosen durch Reizung der Blutgefäßwand (z.B. mittels Laserstrahl oder elektrisch bzw. mechanisch) gehört, am Menschen nicht angewendet werden. Zur genauen klinischen Bewertung von vasoaktiven Medikamenten is es daher erforderlich, Langzeitbeobachtungen an einer großen Anzahl von thrombosegefährdeten Patienten (z.B. Atherosklerose-Patienten, oder Patienten nach Infarkt oder Gefäßoperationen) hinsichtlich auftretender thrombotischer Ereignisse durch zuführen.

Bei Versuchen mit Humanblut, das aus den Venen von Freiwilligen gezogen worden war, wurde nun festgestellt, daß die Hemmung der Thrombozytenaggregation stärker war, wenn dem Blut (bzw. Plasma) der vorher mit vasoaktiven Medikamenten, wie Xanthinen (z.B. Pentoxifyllin) oder Pyrimido-Pyrimidinen oder anderen Hemmstoffen der Phosphodiesterase, behandelten Blutspender ex vivo noch der Gefäßwandmetabolit $PGI_2$ zugesetzt wurde. Dieser Effekt war umso überraschender, als er sogar durch solche $PGI_2$-Konzentrationen, die für sich keine Hemmung der Thrombozytenaggregation bewirken, erzielt werden konnte. Noch überraschender war es nun, daß durch nachträglichen Zusatz ex vivo von $PGI_2$ und eines Cyclooxygenase-Hemmers, wie Acetylsalicylsäure, eine zusätzliche drastische Hemmung der Thromobytenaggregation beobachtet werden konnte, die weit über die hinausging, die durch Zusatz des Cyclooxygenase-Inhibitors alleine, also ohne $PGI_2$, erreicht werden konnte. Die erforderlichen Blut- bzw. Plasmaspiegel an Acetylsalicylsäure können nicht nur durch (den) nachträglichen Zusatz dieses Medikaments ex vivo erhalten werden, sondern können auch durch eine vorherige Behandlung der Versuchsperson mit diesem Medikament, z.B. per os im Blut enthalten sein. Umgekehrt kann das Blut bzw. Plasma auch direkt in vitro mit Phosphodiesterase-Hemmern versetzt werden.

Überraschenderweise läßt sich $PGI_2$ hier auch durch andere Prostaglandine, wie $PGE_1$, $PGD_2$ oder deren Abkömmlinge ersetzen. Dies ist von großem Vorteil; denn diese Prostaglandine, insbesondere $PGE_1$ und $PGD_2$, sind chemisch stabiler als $PGI_2$, das bei physiologischem pH innerhalb von Minuten durch Hydrolyse biologisch inaktiv wird.

Erfindungsgegenstand ist somit ein Verfahren zum Nachweis der antiaggregatorischen Wirkung von Phosphodiesterase-Hemmern (A) und/oder Cyclooxygenase-Hemmern (B) außerhalb des menschlichen oder tierischen Körpers durch

- Zusatz des zu prüfenden Phosphodiesterase-Hemmers (A) und/oder Cyclooxygenase-Hemmers (B) zu dem entnommenen Blut - falls darin nicht schon infolge einer vorherigen Applikation in unveränderter oder in metabolisierter Form vorhanden,
- gegebenenfalls Abtrennung der Erythrozyten und Leukocyten sowie
- Auslösung - vorzugsweise durch Zugabe eines Aggregations-Induktors - und Messung der Thrombozyten-Aggregation;

das Verfahren ist dadurch gekennzeichnet, daß man vor oder spätestens bei der Auslösung der Thrombozyten-Aggregation noch einen Phosphodiesterase-Hemmer (A) - falls bis dahin nur ein Cyclooxygenase-Hemmer (B) zugesetzt wurde -
oder noch einen Cyclooxygenase-Hemmer (B) - falls bis dahin nur ein Phosphodiesterase-Hemmer (A) zugesetzt wurde -
und mindestens ein Prostaglandin (C) zugibt, so daß die Auslösung und Messung der Thrombozyten-Aggregation in Gegenwart der Dreierkombination Phosphodiesterase-Hemmer (A), Cyclooxygenase-Hemmer (B), in der jeweils unveränderten oder in metabolisierter Form, und
Prostaglandin (C) erfolgt.

Aus der bereits bekannten überadditiven antiaggregatorischen Wirkung von Zweierkombinationen, bestehend aus bestimmten Phosphodiesterase-Hemmern (Xanthinen) bzw. dem Cyclooxygenase-Hemmer Acetylsalicylsäure und bestimmten Heteroiminoprostacyclinen ist schon gefolgert worden, daß auch ein Medikament, bestehend aus der Dreierkombination aus bestimmten Xanthinen, der Acetylsalicylsäure und bestimmten Heteroiminoprostacyclinen therapeutisch nützlich sein kann (vgl. DE-A-35 24 051). Der - verglichen mit den jeweiligen Zweierkombinationen - noch stärkere überadditive antiaggregatorische Effekt der Dreierkombination aus Phosphodiesterase-Hemmer, Cyclooxygenase-Hemmer und Prostaglandin wurde jedoch erst jetzt erkannt und ist die Basis der vorliegenden Erfindung.

Der Wert des erfindungsgemäßen Verfahrens besteht in erster Linie darin, daß wegen der hier geschaffenen Möglichkeit des Nachweises der antiaggregatorischen Wirkung von Phosphodiesterase-Hemmern und Cyclooxygenase-Hemmern außerhalb des menschlichen oder tierischen Körpers aufwendige und komplizierte Versuchsreihen am Menschen und am Tier überflüssig gemacht wurden bzw. werden. Die Erfindung wurde mit Pentoxifyllin als Phosphodiesterase-Hemmer und mit Acetylsalicylsäure als Cyclooxygenase-Hemmer ausgearbeitet, deren antiaggregatorische Wirkung bekannt ist; die Anwendung auf andere Phosphodiesterase-Hemmer und Cyclooxygenase-Hemmer funktioniert jedoch in gleicher Weise und läßt dann auch einen Vergleich etwa zur antiaggregatorischen Wirkung von Pentoxifyllin und Acetylsalicylsäure zu (siehe den experimentellen Teil).

Das Verfahren ist im Prinzip mit allen möglichen Phosphodiesterase-Hemmern, Cyclooxygenase-Hemmern und Prostaglandinen durchfuhrbar; bevorzugt ist jedoch die Durchführung mit folgenden Verbindungen:

Phosphodiesterase-Hemmer (A):

Verbindungen mit Xanthinstruktur oder mit Pyrimido-Pyrimidinstruktur; hauptsächlich

a) Xanthinderivate der in der DE-A-35 08 097 beschriebenen Art, d.s. Verbindungen der Formel I

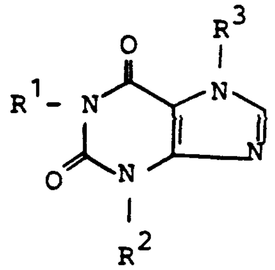

(I)

worin einer der Reste $R^1$ und $R^3$ eine geradkettige Alkyl-, ($\omega$-1)-Oxyalkyl- oder ($\omega$-1)-Hydroxyalkylgruppe mit 3 bis 8 C-Atomen
und die beiden anderen Reste $R^2$ und $R^3$ oder $R^1$ und $R^2$ geradkettige oder verzweigte Alkylgruppen mit 1 bis 8 C-Atomen in der Position von $R^1$ und $R^3$ und mit 1 bis 4 C-Atomen in der Position von $R^2$ darstellen, wobei die Summe der C-Atome dieser beiden Alkylsubstituenten höchstens 10 beträgt,

b) Verbindungen der Formel I mit $R^1$, $R^2$, $R^3$ = unabhängig voneinander H oder $C_1$-$C_4$-Alkyl (d.i. also das unsubstituierte Xanthin, Coffein, Theobromin, Theophyllin, etc.)

c) Xanthinderivate der in der DE-A-35 08 097 beschriebenen Art der Formel Ia:

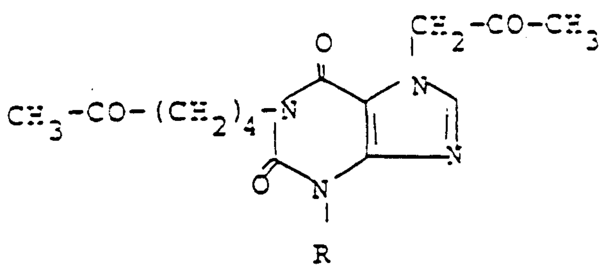

(Ia),

worin R = $C_1$-$C_4$-Alkyl,

d) Salze der unter a, b und c genannten Verbindungen,

e) Pyrimido-Pyrimidine der in der DE-A-35 15 874 genannten Art der Formel III:

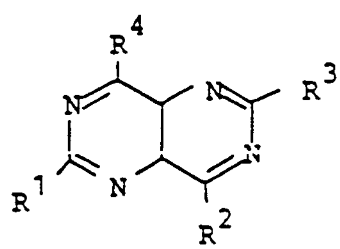

(III),

worin wenigstens einer der Reste $R^1$ und $R^3$ = $N(CH_2$-$CHR^5$-$OH)_2$
und $R^5$ = H oder $CH_3$, und wenigstens einer der Reste $R^2$ und $R^4$ =

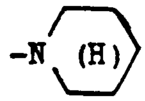

oder =

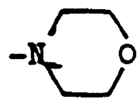

ist und

f) Salze der Verbindungen der Formel III

Cyclooxygenase-Hemmer (B):

O-Acetylsalicylsäure,

| | |
|---|---|
| Indomethacin | (= Nr. 4852 in "The Merck-Index", Rahway/USA, 10th edition 1983) |
| Metamizol | (= Nr. 3369 in dem vorerwähnten Merck-Index) und |
| Ibuprofen | (= Nr. 4797 in dem vorerwähnten Merck-Index) sowie deren Salze. |

Prostaglandine (C):

die in der DE-A-35 26 362, Seite 3, Zeilen 21 - 59 genannten Verbindungen,
speziell Prostaglandin $E_1$, 6-Keto-$PGE_1$, $PGI_2$ und $PGD_2$ sowie deren Salze und Derivate.

Besonders bevorzugte Phosphodiesterase-Hemmer (A), Cyclooxygenase-Hemmer (B) und Prostaglandine (C) sind:

Phosphodiesterase-Hemmer (A):

Pentoxifyllin (= Verbindung der Formel I mit $R^1$ = $-(CH_2)_4-CO-CH_3$ und $R^2 = R^3 = CH_3$),

Dipyridamol (= Verbindung der Formel III mit $R^1 = R^3 = -N(CH_2-CH_2OH)_2$ und $R^2 = R^4$ =

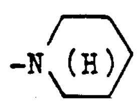 )

Mopidamol (= Verbindung der Formel III mit $R^1 = R^3 = -N(CH_2-CH_2OH)_2$ $R^2$ = H und $R^4$ =

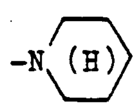 ),

sowie die Salze der beiden letztgenannten Verbindungen.

Cyclooxygenase-Hemmer (B):

O-Acetylsalicylsäure

Prostaglandine (C):

Prostaglandin $E_1$ und 6-Keto-Prostaglandin $E_1$.

Die zu untersuchenden Phosphodiesterase-Hemmer (A) und Cyclooxygenase-Hemmer (B) können dem menschlichen oder tierischen Körper einzeln oder in Kombination - z.B. per os - schon appliziert worden sein oder werden nach der Blutentnahme dem entnommenen Blut zugesetzt.

Selbstverständlich können die Stoffe auch in Prodrug-Form, in Form biologisch aktiver Metaboliten oder als Salze dem Blut zugesetzt werden. Wenn die Salze nach der Blutentnahme dem entnommenen Blut zugesetzt werden, brauchen sie nicht mehr physiologisch unbedenklich sein.

Die Zugabe mindestens eines Prostaglandins (C) erfolgt normalerweise nach der Blutentnahme. Es ist nur immer darauf zu achten, daß vor oder spätestens bei der Auslösung der Thrombozyten-Aggregation die Dreierkombination aus Phosphodiesterase-Hemmer (A), Cyclooxygenase-Hemmer (B) in der jeweils unveränderten oder in metabolisierter Form, und Prostaglandin (C) vorhanden ist.

Dabei arbeitet man bevorzugt in folgenden Konzentrationsbereichen:

| | |
|---|---|
| Phosphodiesterase-Hemmer (A): | ca. $10^{-8}$ bis $10^{-3}$ Mol/l, insbesondere ca. $10^{-6}$ bis $10^{-4}$ Mol/l; |
| Cyclooxygenase-Hemmer (B): | gleicher Konzentrationsbereich wie für (A); |
| Prostaglandin (C): | etwa $10^{-10}$ bis $10^{-6}$ Mol/l, insbesondere etwa $10^{-9}$ bis $10^{-7}$ Mol/l. |

Die hier angegebenen Konzentrationsbereiche sind im wesentlichen nur orientierend. Je nach der Wirksamkeit der einzelnen Substanzen kann es zweckmäßig sein, sich mehr im unteren (bei hoher Wirksamkeit) oder mehr im oberen Konzentrationsbereich (bei schwächerer Wirksamkeit) zu bewegen.

Die Auslösung der Aggregations-Induktion und die Messung der Thrombozyten-Aggregation erfolgen nach an sich bekannten Methoden. Man kann also z.B. zuerst die Erythrocyten und Leukozyten durch Zentrifugieren abtrennen und die Thrombozyten-Aggregation dann im verbleibenden Blutplasma auslösen (mechanisch oder vorzugsweise durch Zugabe eines Aggregations-Induktors wie Arachidonsäure oder Collagen) und optisch messen,
oder man kann die Aggregations-Induktion auch im Vollblut vornehmen und die Aggregation darin dann etwa elektrisch messen.

Bevorzugt ist die chemische Auslösung der Thrombozyten-Aggregation mit Arachidonsäure oder Collagen und die optische Messung der Aggregation im Blutplasma.

## Experimenteller Teil

### Methodik

### Induzierte Aggregation von Humanthrombozyten in vitro - ex vivo

Gesund erscheinenden männlichen und weiblichen Freiwilligen, die im vorangegangenen Zeitraum von 10 Tagen keine Medikamente eingenommen haben (Ausnahme siehe Beispiel 1) wird durch vorsichtige Kanülierung der Antekubitalvene Blut entnommen, das sofort mit Natriumzitrat (ad 0,38 %) stabilisiert wird. Durch 15minütiges Zentrifugieren bei 140 x g (g = Erdbeschleunigung) erhält man im Überstand plättchenreiches Plasma (PRP), dessen Thrombozytengehalt im Bereich 2,5 - 3,5 x $10^8$/ml (Coulter Counter) liegen soll. Die Plättchenaggregation wird optisch durch Messung der Lichttransmission im Born'schen Aggregometer verfolgt. Das Gesamtvolumen des Testansatzes beträgt 0,25 ml. Die Vorinkubationszeit mit dem Prüfpräparat beträgt 10 min bei 37 °C, die Aggregationsinduktion erfolgt sodann mit 2 • $10^{-4}$ Mol/l Arachidonsäure oder 2,4 µg/ml Collagen. Die Prüfpräparate werden an jeweils fünf verschiedenen Spendern getestet. Aus den jeweiligen maximalen Aggregationsamplituden werden Dosis-Wirkungskurven erstellt und graphisch die arithmetisch gemittelten $ED_5$ (Mol/l)-Werte bestimmt ($ED_{50}$ = die für 50 %ige Hemmwirkung erforderliche Konzentration).

### Beispiel 1

### 1. Humanstudie ex vivo:

Einer freiwilligen Versuchsperson wurden antecubital 25 ml Blut entnommen und daraus auf üblichem Wege (siehe Methodik) Zitratplasma für die Kontroll(vergleichs-)messungen hergestellt.

Unmittelbar nach Blutentnahme wurde die Versuchsperson per os mit 600 mg Pentoxifyllin (Trental (R) 600, Firma Albert Roussel Pharma GmbH, Wiesbaden) behandelt. 80 Minuten später wurden wieder 25 ml Blut entnommen, und daraus, wie oben beschrieben, Zitratplasma für den Verumversuch hergestellt. Nach Inkubation der Proben bei 25 °C wurden Kontroll- und Verumplasma jeweils mit Collagen versetzt und, wie oben beschrieben, die Thrombozyten-Aggregation gemessen (Kontrollversuch siehe Tabelle 1, Exp. 1). Sodann wurde das jeweilige Plasma mit 5,5 x $10^{-8}$ Mol/l $PGE_1$, bzw. mit 1,1 x $10^{-5}$ Mol/l O-Acetylsalicylsäure versetzt (Inkubationszeiten s. Tabelle, in Klammern). Auch in diesem Fall konnte eine praktisch ungehinderte Thrombozyten-Aggregation ausgelöst werden (Exp. II und III). Acetylsalicylsäure und $PGE_1$, die zwar an sich als Inhibitoren der Thrombozyten-Aggregation bekannt sind, bewirken in diesen Konzentrationen praktisch keine Hemmung, d.h. es wird vollständige (= 0 % Hemmung der Aggregation) oder fast vollständige Aggregation gemessen.

**Ergebnisse:**

**A) Kontrollversuch** (Versuchsperson unbehandelt)

| Experiment | | Hemmung der Aggregation |
|---|---|---|
| I | ohne Medikamentenzusatz ex vivo | 0 % |
| II | $1,1 \cdot 10^{-5}$ Mol/l Acetylsalicylsäure (10 Min) | 2 % |
| III | $5,5 \cdot 10^{-8}$ Mol/l Prostaglandin $E_1$ (15 Min) | 12 % |

Ergebnisse: (Fortsetzung)

| Experiment | A) **Kontrollversuch** (Versuchsperson unbehandelt) | Hemmung der Aggregation |
|---|---|---|
| IV | $1{,}1 \cdot 10^{-5}$ Mol/l Acetylsalicylsäure (10 Min) + $5{,}5 \cdot 10^{-8}$ Mol/l Prostaglandin $E_1$ (15 Min) | 63 % |
| V | $7{,}2 \cdot 10^{-5}$ Mol/l Pentoxifyllin (25 Min) | 0 % |
| VI | $7{,}2 \cdot 10^{-5}$ Mol/l Pentoxifyllin (25 Min) + $5{,}5 \cdot 10^{-8}$ Mol/l Prostaglandin $E_1$ (15 Min) | 21 % |
| | B) **Verumversuch** (Pentoxifyllin-behandelte Versuchsperson) | |
| VII | ohne Zusätze | 4 % |
| VIII | $1{,}1 \cdot 10^{-5}$ Mol/l Acetylsalicylsäure (10 Min) | 23 % |

Ergebnisse: (Fortsetzung)

| Experiment | B) **Verumversuch** (Pentoxifyllin-behandelte Versuchsperson) | Hemmung der Aggregation |
|---|---|---|
| IX | $5{,}5 \cdot 10^{-8}$ Mol/l Prostaglandin $E_1$ (15 Min) | 27 % |
| X | $1{,}1 \cdot 10^{-5}$ Mol/l Acetylsalicylsäure (10 Min) + $5{,}5 \cdot 10^{-8}$ Mol/l Prostaglandin $E_1$ (15 Min) | 91 % |

**(Alle Meßwerte sind auf Exp. I = 0 % Hemmung der Aggregation, d.h. vollständige Aggregation, bezogen)**

Insbesondere ein Vergleich des Experiments I mit VII zeigt, daß die Aggregation ex vivo nach oraler Behandlung der Versuchsperson mit Pentoxifyllin nur in geringem Ausmaß verändert ist. Der hemmende Effekt von Pentoxifyllin auf die Aggregation ex vivo wird jedoch deutlich, wenn dem Thrombozytensystem ex vivo $PGE_1$ zugesetzt wird (Exp. III vs. IX). Noch stärker wird der Hemmeffekt (vgl. Exp. IV vs. X), wenn ex vivo $PGE_1$ und Acetylsalicylsäure zugesetzt wird.

Ähnliche antiaggregatorische Wirkungen ex vivo am Menschen werden erhalten, wenn die mit Pentoxifyllin per os behandelte Versuchsperson ebenfalls per os mit Acetylsalicylsäure behandelt wird, und das Blutplasma vor der Bestimmung der Thrombozytenaggregation ex vivo mit $PGE_1$ versetzt wird.

Die nachfolgend beschriebenen Aggregationsexperimente wurden in vitro mit Humanplasma durchgeführt, wie unter "Methodik" beschrieben.

Vor der Aggregationsinduktion wurde das Plasma in vitro mit dem Phosphodiesterase-Hemmer (A) 20 Min., mit dem Cyclooxygenase-Hemmer (B) 10 Min. und mit dem Prostaglandin (C) 25 Min. inkubiert. Die Vergleichsexperimente gemäß dem Stand der Technik wurden mit jeweils einer bzw. mit zweien der genannten Wirkstoffklassen entsprechend durchgeführt. (Die Bezifferung der Experimente ist von derjenigen auf den vorhergehenden Seiten unabhängig).

| | | % Hemmung der Aggregation durch Monosubstanzen |
|---|---|---|
| **Phosphodiesterase-Hemmer (A)** | | |
| VI | $5,3 \cdot 10^{-5}$ mol/l Dipyridamol | 3 |
| VII | $1,0 \cdot 10^{-5}$ mol/l Mopidamol | 4 |
| VIII | $7,2 \cdot 10^{-5}$ mol/l Pentoxifyllin | 0 |
| IX | $1,2 \cdot 10^{-4}$ mol/l Pentoxifyllin | 5 |
| X | $2,0 \cdot 10^{-4}$ mol/l Pentifillin | 7 |
| XI | $2,0 \cdot 10^{-4}$ mol/l Propentofyllin (= 1-(5'-Oxohexyl)-3-methyl-7-propyl-xanthin) | 6 |
| XII | $7,5 \cdot 10^{-5}$ mol/l 3,7-Dimethyl-1-(3-carboxypropyl)xanthin (Metabolit von Pentoxifyllin) | 2 |
| XIII | $7,5 \cdot 10^{-5}$ mol/l 3,7-Dimethyl-1-(5-hydroxyhexyl)xanthin (Metabolit von Pentoxifyllin | 0 |
| XIV | $4,4 \cdot 10^{-4}$ mol/l Theophyllin | 8 |

**Cyclooxygenase-Hemmer (B)**

| | | | |
|---|---|---|---|
| I | $1{,}1\cdot10^{-5}$ mol/l | Acetylsalicylsäure | 0 |
| II | $5{,}5\cdot10^{-5}$ mol/l | Acetylsalicylsäure | 15 |
| III | $5{,}6\cdot10^{-7}$ mol/l | Indomethacin | 0 |
| IV | $9{,}2\cdot10^{-7}$ mol/l | Methylaminoantipyrin (Metabolit von Metamizol) | 2 |
| V | $9{,}7\cdot10^{-7}$ mol/l | Ibuprofen | 5 |

**Prostaglandine (C)**

| | | | |
|---|---|---|---|
| XV | $2{,}0\cdot10^{-10}$ mol/l | $PGI_2$ | 0 |
| XVI | $2{,}0\cdot10^{-8}$ mol/l | $PGE_1$ | 7 |
| XVII | $5{,}0\cdot10^{-8}$ mol/l | $PGE_1$ | 10 |

**% Hemmung der Aggregation**

| durch Kombinationen gemäß Stand der Technik | | durch erfindungsgemäße Kombinationen | |
|---|---|---|---|
| I + VI | 3 | I + VI + XV | 89 |
| I + VII | 5 | I + VII + XVI | 92 |
| I + VIII | 1 | I + VIII + XVI | 87 |
| I + IX | 5 | I + IX + XVI | 91 |
| I + X | 8 | I + X + XVII | 95 |
| I + XI | 7 | I + XI + XV | 91 |
| I + XII | 3 | I + XII + XVI | 92 |
| I + XIII | 2 | I + XIII + XV | 85 |
| I + XIV | 10 | I + XIV + XVII | 97 |
| I + XV | 10 | | |
| I + XVI | 10 | | |
| I + XVII | 13 | | |
| II + VI | 18 | II + VI + XVI | 88 |
| II + IX | 22 | II + IX + XVI | 89 |
| II + XVI | 31 | | |
| III + VI | 3 | III + VI + XVI | 87 |
| III + VIII | 2 | III + VIII + XVI | 85 |
| III + XVI | 11 | | |

% Hemmung der Aggregation (Fortsetzung)

| durch Kombinationen gemäß Stand der Technik | | durch erfindungsgemäße Kombinationen | |
|---|---|---|---|
| IV + VIII | 2 | IV + VIII + XVI | 82 |
| IV + XVI | 12 | | |
| V + VII | 10 | V + VII + XV | 84 |
| V + IX | 7 | V + IX + XVII | 87 |
| V + XV | 11 | | |
| V + XVII | 18 | | |
| VI + XV | 5 | | |
| VII + XVI | 15 | | |
| IX + XVI | 16 | | |
| XIV + XVII | 21 | | |
| XI + XV | 9 | | |

## Patentansprüche

**1.** Verfahren zum Nachweis der antiaggregatorischen Wirkung von Phosphodiesterase-Hemmern (A) und/oder Cyclooxygenase-Hemmern (B) außerhalb des menschlichen oder tierischen Körpers durch

- Zusatz des zu prüfenden Phosphodiesterase-Hemmers (A) und/oder Cyclooxygenase-Hemmers (B) zu dem entnommenen Blut - falls darin nicht schon infolge einer vorherigen Applikation in unveränderter oder in metabolisierter Form vorhanden,
- gegebenenfalls Abtrennung der Erythrozyten und Leukozyten sowie
- Auslösung -vorzugsweise durch Zugabe eines Aggregationsinduktors - und Messung der Thrombozyten-Aggregation,

dadurch gekennzeichnet, daß man vor oder spätestens bei der Auslösung der Thrombozyten-Aggregation noch einen Phosphodiesterase-Hemmer (A) - falls bis dahin nur ein Cyclooxygenase-Hemmer (B) zugesetzt wurde -

oder noch einen Cyclooxygenase-Hemmer (B) - falls bis dahin nur ein Phosphodiesterase-Hemmer (A) zugesetzt wurde -

und mindestens ein Prostaglandin (C) zugibt, so daß die Auslösung und Messung der Thrombozyten-Aggregation in Gegenwart der Dreierkombination Phosphodiesterase-Hemmer (A), Cyclooxygenase-Hemmer (B), in der jeweils unveränderten oder in metabolisierter Form, und
Prostaglandin (C) erfolgt.

**2.** Verfahren nach Anspruch 1, gekennzeichnet durch den Einsatz von Verbindungen mit Xanthin-Struktur oder mit Pyrimido-Pyrimidin-Struktur als Phosphodiesterase-Hemmer (A).

**3.** Verfahren nach Anspruch 1 oder 2, gekennzeichnet durch den Einsatz folgender Verbindungen als Phosphodiesterase-Hemmer (A), Cyclooxygenase-Hemmer (B) und Prostaglandine (C):

Phosphodiesterase-Hemmer (A):
a) Xanthinderivate der Formel I

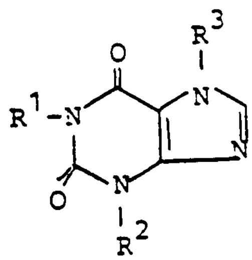

(I)

worin einer der Reste $R^1$ und $R^3$ eine geradkettige Alkyl-, ($\omega$-1)-Oxyalkyl- oder ($\omega$-1)-Hydroxyalkyl-Gruppe mit 3 bis 8 C-Atomen
und die beiden anderen Reste $R^2$ und $R^3$ oder $R^1$ und $R^2$ geradkettige oder verzweigte Alkylgruppen mit 1 bis 8 C-Atomen in der Position von $R^1$ und $R^3$ und mit 1 bis 4 C-Atomen in der Position von $R^2$ darstellen, wobei die Summe der C-Atome dieser beiden Alkylsubstituenten höchstens 10 beträgt,
b) Verbindungen der Formel I mit $R^1$, $R^2$, $R^3$ = unabhängig voneinander H oder $C_1$-$C_4$-Alkyl,
c) Xanthinderivate der Formel Ia

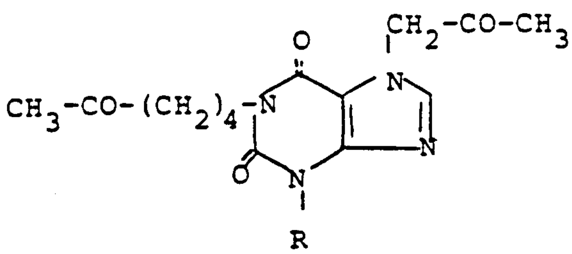

(Ia)

worin R = $C_1$-$C_4$-Alkyl,
d) Salze der unter a, b und c genannten Verbindungen,
e) Pyrimido-Pyrimidine der Formel III

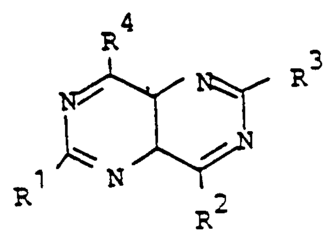

(III)

worin wenigstens einer der Reste $R^1$ und $R^3$ = $-N(CH_2-CHR^5-OH)_2$ und $R^5$ = H oder $CH_3$, und wenigstens einer der Reste $R^2$ und $R^4$ =

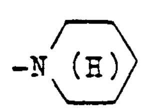

oder

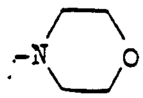

ist und

f) Salze der Verbindungen der Formel III,

Cyclooxygenase-Hemmer (B):
O-Acetylsalicylsäure,
Indomethacin,
Metamizol und
Ibuprofen
sowie deren Prodrug-Formen, biologisch aktive Metabolite und Salze,

Prostaglandine (C):
Prostaglandin (PG)$E_1$, 6-Keto-$PGE_1$, $PGI_2$ und $PGD_2$
sowie deren Salze und Derivate.

**4.** Verfahren nach einem oder mehreren der Ansprüche 1 bis 3, gekennzeichnet durch den Einsatz folgender Verbindungen als
Phosphodiesterase-Hemmer (A), Cyclooxygenase-Hemmer (B) und Prostaglandine (C):
Phosphodiesterase-Hemmer (A):
Pentoxifyllin (= Verbindung der Formel I mit $R^1$ = $-(CH_2)_4-CO-CH_3$ und $R^2$ = $R^3$ = $CH_3$),
Dipyridamol (= Verbindung der Formel III mit $R^1$ = $R^3$ = $-N(CH_2-CH_2OH)_2$ und $R^2$ = $R^4$ =

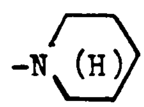

)

Mopidamol (= Verbindung der Formel III mit $R^1$ = $R^3$ = $-N(CH_2-CH_2OH)_2$ $R^2$ = H und $R^4$ =

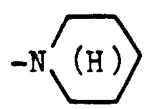

,

sowie die Salze der beiden letztgenannten Verbindungen;
Cyclooxygenase-Hemmer (B):
O-Acetylsalicylsäure;

Prostaglandine (C): Prostaglandin $E_1$ und 6-Keto-Prostaglandin $E_1$.

**5.** Verfahren nach einem oder mehreren der Ansprüche 1 bis 3, gekennzeichnet durch die Einstellung folgender Konzentrationen:

| | |
|---|---|
| Phosphodiesterase-Hemmer (A): | ca. $10^{-8}$ bis $10^{-3}$ Mol/l, insbesondere ca. $10^{-6}$ bis $10^{-4}$ Mol/l; |
| Cyclooxygenase-Hemmer (B): | gleicher Konzentrationsbereich wie für den Phosphodiesterase-Hemmer (A); |
| Prostaglandin (C): | etwa $10^{-10}$ bis $10^{-6}$ Mol/l, insbesondere etwa $10^{-9}$ bis $10^{-7}$ Mol/l. |

**6.** Verfahren nach einem oder mehreren der Ansprüche 1 bis 5, dadurch gekennzeichnet, daß man die Thrombozyten-Aggregation chemisch auslöst und als Aggregations-Induktor Arachidonsäure oder Collagen verwendet.

**7.** Verfahren nach einem oder mehreren der Ansprüche 1 bis 6, dadurch gekennzeichnet, daß man die Thrombozyten-Aggregation optisch im Blutplasma mißt.

## Claims

1. A method for detecting the antiaggregatory effect of phosphodiesterase inhibitors (A) and/or cyclooxygenase inhibitors (B) outside the human or animal body by
   - addition of the phosphodiesterase inhibitor (A) and/or cyclooxygenase inhibitor (B) which is to be tested to the blood sample - if not already present therein, in unchanged or in metabolized form, as a consequence of previous administration,
   - where appropriate removal of the erythrocytes and leukocytes and
   - initiation - preferably by addition of an aggregation inducer - and measurement of the platelet aggregation,

   which comprises a phosphodiesterase inhibitor (A) also being added - if only a cyclooxygenase inhibitor (B) has been added up till then - or a cyclooxygenase inhibitor (B) also being added - if only a phosphodiesterase inhibitor (A) has been added up till then - and at least one prostaglandin (C) being added, before, or no later than at, the initiation of the platelet aggregation, so that the initiation and measurement of the platelet aggregation takes place in the presence of the ternary combination of
   phosphodiesterase inhibitor (A),
   cyclooxygenase inhibitor (B),
   each in the unchanged or in metabolized form, and prostaglandin (C).

2. The method as claimed in claim 1, wherein compounds with a xanthine structure or with a pyrimido-pyrimidine structure are used as phosphodiesterase inhibitors (A).

3. The method as claimed in claim 1 or 2, wherein the following compounds are used as phosphodiesterase inhibitors (A), cyclooxygenase inhibitors (B) and prostaglandins (C):
   Phosphodiesterase inhibitors (A):
   a) xanthine derivatives of the formula I

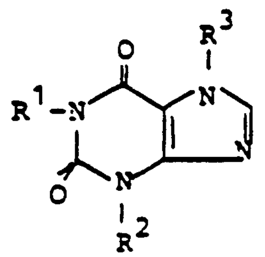

(I)

   in which one of the radicals $R^1$ and $R^3$ represents a straight-chain alkyl, ($\omega$-1)-oxyalkyl or ($\omega$-1)-hydroxyalkyl group having 3 to 8 carbon atoms, and the other two radicals $R^2$ and $R^3$ or $R^1$ and $R^2$ represent straight-chain or branched alkyl groups having 1 to 8 carbon atoms in the position of $R^1$ and $R^3$ and having 1 to 4 carbon atoms in the position of $R^2$, with the total of the carbon atoms in these two alkyl substituents not exceeding 10,
   b) compounds of the formula I with $R^1$, $R^2$ and $R^3$ being, independently of one another, H or $C_1$-$C_4$-alkyl,
   c) xanthine derivatives of the formula Ia

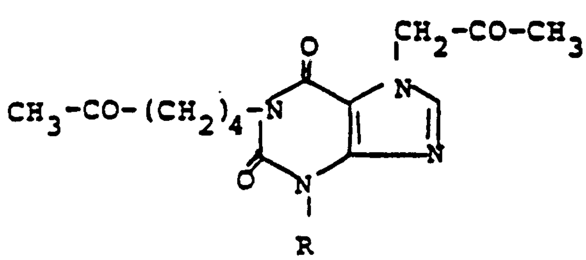

(Ia)

   in which R = $C_1$-$C_4$-alkyl,
   d) salts of the compounds mentioned under a, b and c,

e) pyrimido-pyrimidines of the formula III

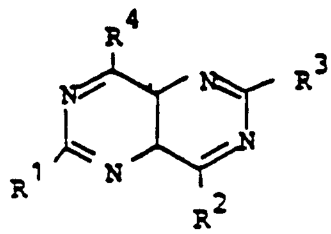

(III)

in which at least one of the radicals $R^1$ and $R^3$ is $-N(CH_2-CHR^5-OH)_2$, and $R^5$ is H or $CH_3$, and at least one of the radicals $R^2$ and $R^4$ is

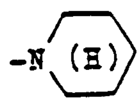

or

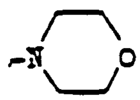

,

and

f) salts of the compounds of the formula III,

Cyclooxygenase inhibitors (B):
O-acetylsalicylic acid,
indomethacin,
metamizole and
ibuprofen,
and the prodrug forms, biologically active metabolites and salts thereof,
Prostaglandins (C):
prostaglandin $(PG)E_1$, 6-keto-$PGE_1$, $PGI_2$ and $PGD_2$, and the salts and derivatives thereof.

**4.** The method as claimed in one or more of claims 1 to 3, wherein the following compounds are used as phosphodiesterase inhibitors (A), cyclooxygenase inhibitors (B) and prostaglandins (C):
Phosphodiesterase inhibitors (A):

pentoxifylline (= compound of the formula I with $R^1 = -(CH_2)_4-CO-CH_3$ and $R^2 = R^3 = CH_3$),

dipyridamole (= compound of the formula III with $R^1 = R^3 = -N(CH_2-CH_2OH)_2$ and $R^2 = R^4 =$

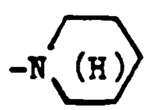

)

mopidamol (= compound of the formula III with $R^1 = R^3 = -N(CH_2-CH_2OH)_2$, $R^2 = H$ and $R^4 =$

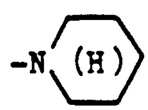

),

as well as the salts of the two latter compounds;
Cyclooxygenase inhibitors (B):
O-acetylsalicylic acid;
Prostaglandins (C):
prostaglandin $E_1$ and 6-keto-prostaglandin $E_1$.

5. The method as claimed in one or more of claims 1 to 3, wherein the following concentrations are set up:

| | |
|---|---|
| Phosphodiesterase inhibitor (A): | about $10^{-8}$ to $10^{-3}$ mol/l, in particular about $10^{-6}$ to $10^{-4}$ mol/l; |
| Cyclooxygenase inhibitor (B): | same concentration range as for phosphodiesterase inhibitor (A); |
| Prostaglandin (C): | about $10^{-10}$ to $10^{-6}$ mol/l, in particular about $10^{-9}$ to $10^{-7}$ mol/l. |

6. The method as claimed in one or more of claims 1 to 5, wherein platelet aggregation is initiated chemically, and arachidonic acid or collagen is used as aggregation inducer.

7. The method as claimed in one or more of claims 1 to 6, wherein the platelet aggregation is measured optically in the blood plasma.

**Revendications**

1. Procédé pour mettre en évidence l'activité antiagrégante d'inhibiteurs de phosphodiestérase (A) et/ou d'inhibiteurs de cyclooxygénase (B) à l'extérieur du corps humain ou animal par
   - addition de l'inhibiteur de phosphodiestérase (A) et/ou de l'inhibiteur de cyclooxygénase (B) à examiner au sang prélevé
   - au cas où ils n'y sont pas déjà présents sous forme non modifiée ou métabolisée à la suite d'une administration préalable,
   - séparation éventuelle des érythrocytes et des leucocytes et
   - déclenchement - de préférence par addition d'un inducteur de l'agrégation - et mesure de l'agrégation des thrombocytes,

   caractérisé en ce que l'on rajoute, avant ou au plus tard au moment du déclenchement de l'agrégation des thrombocytes, un inhibiteur de phosphodiestérase (A) - au cas où jusqu'alors seul un inhibiteur de cyclooxygénase (B) a été ajouté - ou un inhibiteur de cyclooxygénase (B) - au cas où jusqu'alors seul un inhibiteur de phosphodiestérase (A) a été ajouté - et au moins une prostaglandine (C),
   de façon que le déclenchement et la mesure de l'agrégation des thrombocytes se fassent en présence de la combinaison ternaire constituée par l'inhibiteur de phosphodiestérase (A), l'inhibiteur de cyclooxygénase (B), chacun d'eux étant sous forme non modifiée ou sous forme métabolisée, et
   la prostaglandine (C).

2. Procédé selon la revendication 1, caractérisé par l'utilisation de composés ayant une structure de xanthine ou une structure de pyrimidopyrimidine comme inhibiteurs de phosphodiestérase (A).

3. Procédé selon la revendication 1 ou 2, caractérisé par l'utilisation des composés suivants comme inhibiteurs de phosphodiestérase (A), inhibiteurs de cyclooxygénase (B) et prostaglandines (C):
   inhibiteurs de phosphodiestérase (A):
   a) des dérivés de xanthine de formule I

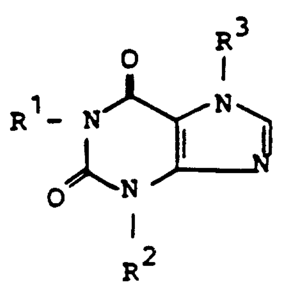

(I)

dans laquelle l'un des restes $R^1$ et $R^3$ représente un groupe alkyle, ($\omega$-1)-oxyalkyle ou ($\omega$-1)-hydroxyalkyle linéaire de 3 à 8 atomes de carbone,
et les deux autres restes $R^2$ et $R^3$ ou $R^1$ et $R^2$ représentent des groupes alkyle linéaires ou ramifiés de 1 à 8 atomes de carbone dans la position de $R^1$ et $R^3$ et de 1 à 4 atomes de carbone dans la position de $R^2$, la somme des atomes de carbone de ces deux substituants alkyle s'élevant au plus à 10,

b) des composés de formule (I) avec $R^1$, $R^2$, $R^3$ = indépendamment l'un de l'autre H ou alkyle en $C_1$-$C_4$,

c) des dérivés de xanthine de formule (Ia)

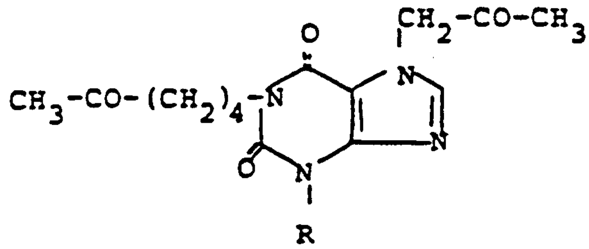

(Ia)

dans laquelle R = alkyle en $C_1$-$C_4$,

d) des sels des composés cités sous a, b, et c,

e) des pyrimidopyrimidines de formule III:

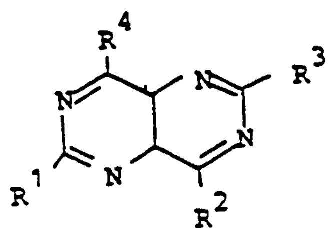

(III)

dans laquelle
au moins l'un des restes $R^1$ et $R^3$ = $N(CH_2\text{-}CHR^5\text{-}OH)_2$ et $R^5$ = H ou $CH_3$,
et au moins l'un des restes $R^2$ et $R^4$ =

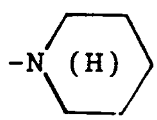 ou 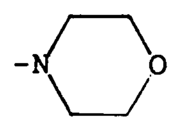

et

f) des sels des composés de formule (III),

inhibiteurs de cyclooxygénase (B):

l'acide o-acétylsalicylique
l'indométhacine
le métamizole et
l'ibuprofène
ainsi que leurs formes de promédicaments, leurs métabolites et leurs sels biologiquement actifs,

prostaglandines (C): la prostaglandine $(PG)E_1$, la 6-céto-$PGE_1$, la $PGI_2$ et la $PGD_2$ et leurs sels et dérivés.

**4.** Procédé selon l'une ou plusieurs des revendications 1 à 3, caractérisé par l'utilisation des composés suivants comme inhibiteurs de phosphodiestérase (A), inhibiteurs de cyclooxygénase (B) et prostaglandines (C):

inhibiteurs de phosphodiestérase (A): la pentoxyfylline (= composé de formule (I) avec $R^1$ = $-(CH_2)_4\text{-}CO\text{-}CH_3$ et $R^2$ = $R^3$ = $CH_3$), le dipyridamole (= composé de formule III avec $R^1$ = $R^3$ = $-N(CH_2\text{-}CH_2OH)_2$ et
$R^2$ = $R^4$ =

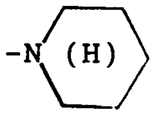 )

le mopidamole (= composé de formule III avec $R^1 = R^3 = -N(CH_2-CH_2OH)_2$, $R^2 = H$ et $R^4 =$

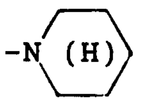 )

et les sels des deux derniers composés cités.

inhibiteurs de cyclooxygénase (B): l'acide o-acétylsalicylique

prostaglandines (C): la prostaglandine $E_1$ et la 6-cétoprostaglandine $E_1$.

**5.** Procédé selon l'une ou plusieurs des revendications 1 à 3, caractérisé par l'établissement des concentrations suivantes:
inhibiteur de phosphodiestérase (A): d'environ $10^{-8}$ à $10^{-3}$ mole/l, en particulier d'environ $10^{-6}$ à $10^{-4}$ mole/l; inhibiteur de cyclooxygénase (B): même domaine de concentrations que pour l'inhibiteur de phosphodiestérase (A);
prostaglandine (C): d'environ $10^{-10}$ à $10^{-6}$ mole/l, en particulier d'environ $10^{-9}$ à $10^{-7}$ mole/l.

**6.** Procédé selon l'une ou plusieurs des revendications 1 à 5, caractérisé en ce qu'on déclenche chimiquement l'agrégation des thrombocytes et qu'on utilise l'acide arachidonique ou le collagène comme inducteur de l'agrégation.

**7.** Procédé selon l'une ou plusieurs des revendications 1 à 6, caractérisé en ce qu'on mesure l'agrégation des thrombocytes dans le plasma sanguin par un moyen optique.